# EUROPEAN PATENT SPECIFICATION

(11) **EP 4 155 880 B1**
(45) Date of publication and mention of the grant of the patent: **23.04.2025**
(21) Application number: 22196975.1
(22) Date of filing: 21.09.2022
(51) Int. Cl.: G06F 3/01, G06N 3/08, G06N 20/00

(54) **A METHOD PERFORMED BY AN INFORMATION PROCESSING DEVICE, A PROGRAM PRODUCT AND A SYSTEM FOR PROCESSING CONTEXT RELATED OPERATIONAL AND HUMAN MENTAL BRAIN ACTIVITY DATA**
VERFAHREN, DAS VON EINER INFORMATIONSVERARBEITUNGSVORRICHTUNG DURCHGEFÜHRT WIRD, PROGRAMMPRODUKT UND SYSTEM ZUR VERARBEITUNG VON KONTEXTBEZOGENEN BETRIEBS- UND MENSCHLICHEN MENTALEN HIRNAKTIVITÄTSDATEN
PROCÉDÉ MIS EN OEUVRE PAR UN DISPOSITIF DE TRAITEMENT D'INFORMATIONS, PRODUIT PROGRAMME ET SYSTÈME DE TRAITEMENT DE DONNÉES D'ACTIVITÉ CÉRÉBRALE FONCTIONNELLE ET MENTALE HUMAINE

(30) Priority: 22.09.2021 NL 1044171
(43) Date of publication of application: 29.03.2023
(73) Proprietor: Zander Laboratories B.V., 2628 XJ Delft (NL)
(72) Inventor: ZANDER, Thorsten, BERLIN (DE); KROL, Laurens, BERLIN (DE)
(74) Representative: Dohmen, Johannes Maria Gerardus

(56) References cited:
- DAVID C JANGRAW ET AL: "Neurally and ocularly informed graph-based models for searching 3D environments", JOURNAL OF NEURAL ENGINEERING, INSTITUTE OF PHYSICS PUBLISHING, BRISTOL, GB, vol. 11, no. 4, 3 June 2014 (2014-06-03), pages 46003, XP020268467, ISSN: 1741-2552, [retrieved on 20140603], DOI: 10.1088/1741-2560/11/4/046003

## Description

### Technical Field

The present application relates to data processing performed and implemented in an information processing device, such as but not limited to a computer, a computing device, a virtual machine, a server, or a plurality of cooperatively operating computers, computing devices, virtual machines, or servers. More specifically, the present application concerns the processing of operational data and human brain activity data by machine learning technology.

### Background

Machine Learning, ML, technology has become indispensable in modern society. Data processing systems using ML technology have proven to handle and complete tasks commonly associated with human beings with more precision and in less time than would be possible for humans.

In general, ML comprises the use and development of computer systems that can learn and adapt without following explicit instructions, by using algorithms and statistical models, including but not limited to those implemented using neural networks, to analyze and draw inferences from patterns or other systematic regularities in data.

Most ML systems presently available are so-called 'tasks intelligent systems', designed to automatically perform singular tasks, focused on highly specific technical domains, such as facial recognition, speech recognition/voice assistants, or searching the internet. Generally referred to as Artificial Narrow Intelligence, ANI, or weak Artificial Intelligence, weak Al.

Such weak or narrow Al algorithms are generally trained through many iterations of their respective task, receiving performance feedback from an evaluation function, or reward function, error function, etc. in order to reinforce or otherwise learn beneficial behavior and/or unlearn or disincentivize actions that do not lead to beneficial behavior or intended outcomes within the given context.

This need for an evaluation function is why many current Al applications are games such as chess or other two-dimensional games, where the final game outcome or continuous game score can serve to evaluate in-game behaviors. Furthermore, games represent a context of limited dimensionality in which the dependencies between Al actions, context parameters, and game outcome or score can be successfully mapped. Finally, the game speed can be increased arbitrarily to allow for more training iterations per unit of time.

While these systems may appear intelligent, they operate under a rather simple set of constraints and limitations not mimicking or replicating human intelligence. These systems merely optimize human behavior as to speed and reliability in performing a single, well-defined task, but do not add real intelligence.

ANI has in recent years reached human-level or even greater performance in specific, well-defined tasks through advances in machine learning, and deep learning in particular, by processing larger amounts of operational or contextual data, among which environmental data, information data, measurement data, control data and state data of a plurality of devices operating in a particular well-defined context or environment.

However, more scenarios, handling multiple, versatile and/or non-predefined tasks, do not have an objective evaluation function like a game score, and cannot arbitrarily be sped up. To interpret actions in more scenarios in terms of goal achievement, benefit, or appropriateness within the current context or a larger strategy, a human is needed to perform that interpretation. Humans can distill relevant dimensions or key factors from situations and evaluate the facts or the events that are taking place accordingly.

Experiments have been performed to impart human intelligence data into the processing of a task intelligent ML system, by human evaluation of a task performed or proposed by the system, such as a game.

This has led to ML training scenarios where human observers press buttons or provide spoken input or otherwise perform explicit ratings to indicate what is appropriate/inappropriate behavior to train an ML algorithm. In this way, by the active feedback or guidance provided by the human user, the system successively learns the best possible policy or policies to perform a respective task.

Human observers may, for example, monitor an ML algorithm traversing a space performing various actions, and explicitly communicate whether or not each change in movement direction or each chosen action is conducive to the goal.

The requirement for these human observers to make explicit decisions concerning what is appropriate or not and to subsequently communicate their judgement, for example by pressing the corresponding button, significantly prolongs training times of the ML algorithm. This situation could be improved by removing the need to press buttons, for example.

In the emerging field of neuroadaptive technologies, patterns of brain activity have been identified in several scientific studies as being associated to internal mental states, for example workload, reflecting interpretations of or responses to the environment, such as error, surprise, agreement, or combinations thereof, in particular human predictive coding, one of the main mechanisms enabling human intelligence. This predictive coding can be understood as an automized judgement function of the human brain, that evaluates all perceived information automatically, according to a subjective/personalized value system. Other mental states, such as agreement or satisfaction, can also be seen as an implicit judgement of perceived information, and may be utilized in a similar function.

Neuroadaptive technology works by identifying low-level data on brain activation, such as event-related potentials, which is then co-registered against the technology's repertoire of possible responses or states. The user's neurophysiological activity in response to known contextual changes is implicitly and continuously monitored, and this activity is cross-referenced in real-time with the changes that caused them. This enables the generative creation of a model that represents the intentions, preferences, interpretations, attitudes, associations, and/or goals of the user.

An example of neuroadaptive technology is disclosed in the paper "Neurally and ocularly informed graph-based models for searching 3D environments", by David C Jangraw et al., published in the Journal of Neural Engineering, Institute of Physics Publishing, Bristol, GB, vol. 11, no. 4, 3 June 2014, page 46003 ff.

In this example electroencephalographic (EEG), saccadic and pupillary data are recorded from subjects as they move through a small part of a 3D virtual city under free-viewing conditions. Using machine learning, the neural and ocular signals evoked by the objects they encounter are integrated and labelled to infer which ones are of subjective interest to them.

These inferred labels are propagated through a large computer vision graph of objects in the city, using semi-supervised learning to identify other, unseen objects that are visually similar to the labeled ones. Finally, the system plots an efficient route to help the subjects visit the 'similar' objects it identifies, resulting in a hybrid brain-computer interface (hBCI) system using naturally evoked ocular signals to improve navigation and information delivery specific to the user's interests.

A tool to assess information about brain activity of an individual is a so-called passive Brain-Computer Interface, pBCI, operating one or more classifiers responsive to the human brain activity.

A pBCI differs significantly from classic Brain-Computer Interfaces, BCls, i.e. active or reactive BCls, which are built on brain activity that is generated or modulated by the operator intentionally to transfer specific control signals to a computer system, thereby replacing other means of input such as a keyboard or computer mouse. pBCI data are based on implicit or passive, involuntary, unintentional, or subconscious human participation, different from explicit or active, i.e. conscious, voluntary, intentional, human interaction with the context.

Specific brain activity has been identified in several scientific studies to adapt or replace an action in a task performed by an ANI ML system, based on a cognitive or affective, i.e. mental, state of a human user involved with and/or monitoring the performance of the task, and/or to learn a model or create a memory of the user responses through an ongoing evaluation of neurophysiological activity of the human user.

A pBCI distinguishes between different cognitive or affective aspects of a human user state, typically recorded through an electroencephalogram, EEG. An immediate action or activity in a context may then be linked to the current mental state or specific aims of a user.

Although at present various ANI systems are available for processing pre-defined and specific tasks in different technical domains, the broader goal is on Al for handling plural tasks in contexts with intelligence proportional to human general intelligence, also known as Artificial General Intelligence, AGI, or strong Al or full Al.

For various reasons it is problematic and difficult to operate machine learning or Al algorithms handling multiple, versatile, and non-predefined tasks when working in a real-life or real-world context. That is a non-predefined or authentic context or environment, occurring in reality or practice, as opposed to an imaginary, a simulated, test or theoretical context, for example.

One problem in real-life contexts is the absence of a finite, a priori repertoire of known responses and states, and the resulting difficulty in identifying which of any number of perceived states bear specific relevance, for example.

The processing, by an ML algorithm or Al of operational data originating in relation to a real-life context or environment for performing context dependent valuations, interpretations, assertions, labelling, etc. presents a further problem, because this type of operations requires interpretation of the context or environment.

For example, paying closer attention to some aspects of the context than to others based on a general understanding of these aspects and their role in the context as a whole, or based on creative insight into the role that these aspects may potentially play. This is a judgement no objective sensor or ML algorithm or Al can make itself, but a human can make such an interpretation.

Those skilled in the art will appreciate that training of machine learning systems for operating in a real-life context and environment by a human user providing active feedback reflecting its interpretations, for example, is practically not feasible because of the large training and calibration data sets involved, making such training very time consuming and demanding for a human user.

Integrating human mental data in the processing of operational data originating in relation to a real-life context or environment, i.e. rendering the system neuroadaptive, presents a further problem because ambiguities and inconsistencies in the relationship between operational data and human mental data of a human participating in the context pose a potential source of error in the data processing of the ML system.

### Summary

It is an object of the present application to provide an improvement to the data processing of computers (information processors) by a method involving a machine learning algorithm capable of operating in a real-life context augmented by human mental brain activity data.

This object is solved by the subject-matter of the independent claims. Preferred embodiments are defined by the dependent claims.

The present method is practically applicable in various real-life scenarios or contexts where humans interact with machines, personal computers, robots, avatars, and many other technical applications.

The expression 'human metal engagement with an aspect of the real-life context', in the light of the present disclosure and the attached claims, refers to what aspect or aspects a human participating with a real-life context - from which operational data originate - is momentarily involved with. The involvement comprised by the expression 'human mental engagement with an aspect of the real-life context' includes both active, conscious or explicit involvement, i.e. the human interacts, is in charge with and/or controls tasks, processes and operations that occur in the real-life context and/or need to be accomplished, and inactive, subconscious or implicit involvement, i.e. when the human is not particularly focused on events, changes, adaptations, modifications etc. that happen in or in relation with the real-life context they nevertheless attract attention, arousal or are otherwise noticed, perceived, or mentally processed by the human.

Mental engagement with an aspect of the real-life context can be sensed from human physiological measurements, that are measurements of the physiological state of a human which can be continuously monitored from pBCI data, operating a respective classifier or classifiers, as well as from measurement data provided by other, different sensors and devices connected to or in data communication with the human, separate from the pBCI.

Investigation by the inventors of the present application has led to the insight that by sensing human mental engagement with an aspect or aspects of a real-life context, plural information assessed from human mental brain activity data provided by a pBCI of a person participating with that real-life context can be tied or linked to operational data originated and acquired from a thus identified aspect or aspects of the real-life context, for processing thereof, independently of the complexity of the real-life context.

Based on the processing of the thus identified operational data and/or human mental brain activity data, i.e. jointly and/or separately, either one or both of the analysis and inferencing by the ML algorithm and aspects of the real-life context are controlled and may be adapted by the information processing device, i.e. the system becomes neuroadaptive. Thereby, among others, enabling context dependent valuations, interpretations, assertions, labelling, etc. by the ML algorithm for operational data acquired from operating in a real-life context, handling multiple, versatile, and non-predefined tasks, acquiring and maintaining repertoires of known states and actions originating from the real-life context and corresponding human mental brain activity, said repertoire thus providing subjective meaning, valuations, interpretations, labels, et cetera, to the and novel operational data, and enabling the prediction of human mental states based on operational data, for example.

For the purpose of the present application and the appended claims, multiple pBCls each operating a different classifier directed to sense different brain activity associated with different types of response or mental states, i.e. mental brain activity data, of a human participant may be used. In practice, tens or hundreds, or more, of classifiers may be deployed.

In accordance with an aspect of the method presented, at least one of the analysis and inferencing by the machine learning algorithm and aspects of the real-life context are controlled by the information processing device by associating the identified operational data and human mental brain activity data.
As such, human mental engagement can inform the ML algorithm which parts of the operational data relate to which mental brain activity data or vice versa, on which basis an association between the two may be established for the purpose of further control and possible adaptation, for example. The establishment of an association can furthermore be used as an identification of the specific relevance of parts of the data, and to enable the above-mentioned context-dependent valuations, interpretations, meaning, etc.

For example, an ML system operating on a battlefield may have any number of context sensors providing operational data to perceive its environment: cameras, thermal imagers, microphones, radar, lidar, chemical composition sensors, seismometers, gyroscopes, etc., and may thus be capable of recording this context and the events taking place within it to any possible degree of objective accuracy.

With the method presented, the ML system now can track which parts of the context the human is consciously and subconsciously attending to. This human may, for example, observe a configuration in the context reminiscent of a trap experienced before. This will automatically and possibly subconsciously evoke a pattern of brain activity that signals the significance of this aspect of the context.

A pBCI can detect this human mental engagement and classify this response and provide it to the ML algorithm, essentially serving as an additional human sensor providing an evaluation of aspects of the environment. The ML algorithm, by the operational data now having available to it a representation of a specific aspect of this real-life context identified through the human's mental engagement with it, and the human's mental evaluation data of this aspect assessed from the identified human mental brain activity data, by combining or associating these pieces of information learns by controlling or adapting its analyzing and inferencing capabilities how to identify, classify, respond to or adapt to different aspects of the real-life context.

In a further aspect of the method presented, at least one of the analysis and inferencing by the machine learning algorithm and aspects of the real-life context are adapted by the information processing device based on at least one of human mental engagement with respect to respective operations and interactions in the real-life context and engagement of the information processing device with respective operations and interactions in said real-life context.

That is, human evaluation and perception of specific or particular, for example predefined, tasks, processes, states, operations and interactions of devices, and environmental information with respect to a real-life context, provided and derived from respective pBCI data, is integrated with the analysis and inferencing of operational or contextual data by the ML algorithm operating in that context.

By incorporating this information into its analyzing and inferencing structure, the ML algorithm not only becomes more intelligent but also learns to mimic the human's preferences, behaviors, interpretations, norms, and values given respective operations and interactions in a real-life context, thus becoming more alike in its own interpretations and actions to the human it learned from.

Autonomous driving vehicles may face decisions with moral dimensions in extreme situations where an accident cannot be avoided, for example. In such situations, the ML system operating the vehicle may need to decide to harm either the human occupant or any of the other road users present at that time. These kinds of decisions could potentially be pre-determined by the manufacturer, opting for example for rule-based behavior to never harm the driver unless it cannot be avoided, and always harm older road users over younger ones.

Such moral rules may, however, vary by culture, with for example Japanese car manufacturers' decisions not necessarily corresponding to those a North American car occupant would want its vehicle to make. Alternatively, the occupant of an autonomous vehicle could configure these decisions themself, for example by answering a large number of questions relating to hypothetical scenarios. Such questions may appear to be impossible to truly answer, however, and such a configuration may additionally need to be repeated more often as attitudes may drift. This furthermore assumes that a corpus of questions can be devised that covers the range of decisions that may be encountered in real life, which is obviously not feasible. Any unforeseen situation may still have to rely on default rule-based behavior that the occupant may not have authorized if given the chance. Ideally, the ML algorithm operating the vehicle would have access to the general moral alignment of its occupant, as well as any momentary moral judgements to inform any immediate decision to be made.

With the present method this can be achieved by monitoring and registering brain activity related to moral interpretations of ongoing situations during driving. The ML system may, for example, as operational data detect the presence of different categories of road users, identify whether or not the human occupant of the vehicle is aware of these road users, i.e. the human's mental engagement with the real-life context, and subsequently extract from the brain activity using a pBCI the moral alignment with respect to these road users.

When driving through a neighborhood, the vehicle may for example pass by playing children, which the vehicle identifies as such through its cameras, and the occupant also recognizes same, as identified by the detection of human mental engagement through the pBCI. Having thus identified the presence of children and the occupant's awareness of them, the pBCI can subsequently be used to identify the occupant's interpretation of these children: an adverse reaction to children or these children in particular can be differentiated from a favorable mental response based on the presence of specific patterns of brain activity.

As such, a natural and otherwise uneventful encounter can be used to implicitly ascertain an element of the occupant's moral values. This can then be used to adjust the ML algorithm's decision process for such scenarios, either in the future or in that very moment, without at any point requiring any explicit communication from the occupant to the vehicle ML system.

Hence, among others, the ML algorithm learns to incorporate moral values, problem solving strategies, preferences, associations, distributions of degrees of acceptance, etc. such that a convergence of human and machine intelligence is initiated and continuously pursued. Over time the ML algorithm learns about the human's subjective interpretations in a bigger scale, building up a profile/model of that human's subjective interpretations.

A striking example of this is a nursing robot in an elderly care facility that has an ML algorithm that allows it to exhibit a range of different behaviors appropriate for the preferences of the respective patients it supports.

As an example, one important dimension here is the degree of autonomy the patient wishes to experience. Depending on this, as well as on various other personal factors, different patients will prefer the robot to perform different amounts and different kinds of tasks. This sentiment may change gradually over time, deviate daily depending on a patient's condition, and may additionally be a function of further situational factors such as visits by family or current actions undertaken by the patient. The robot, tasked to support the patients as best it can, is thus operating in a highly complex and dynamic environment where many of its own operational goals depend on the patient's general or momentary preferences.

When the nursing robot decides to perform a specific action, the patient may interpret this in various ways: for example, they may or may not appreciate it, feel it is in error, or feel guilty for not having performed the task themself. Whenever the robot performs or begins to perform a specific task, it can ensure the patient is aware of its actions by monitoring the patient's mental engagement with itself or the object of its actions. The robot may for example track the patient's eye gaze to itself, may be connected to other devices tracking the patient's eye gaze, identify the patient's attention-directing movements through motion tracking happening at the same time the robot begins the task, may perceive and interpret an auditory reaction from the patient, may itself make a noise or perform another action such as to ensure the patient's awareness, or may ascertain the patient's engagement from patterns of brain activity dependent on the events taking place.

When the patient is aware of the robot's actions, the patient's interpretation of those actions may be implicitly derived from the patient's brain activity using a pBCI. Upon perceiving the robot's actions, the patient's brain activity may exhibit certain patterns which can be meaningfully classified and interpreted by the robot as a favorable or unfavorable interpretation of its actions. The corresponding variable spatiotemporal patterns of brain activity may occur once following (mental engagement with) a specific action or may accompany the action throughout to various degrees.

The robot can furthermore sense additional situational parameters such as the presence and potentially the identity of other persons in the environment, the time of day, the time since a last meal/medication/bathroom visit, the temperature, humidity, etc. Based on this, the robot can learn that, given the current context as expressed through the sensed situational or operational parameters, the patient mentally responds in the perceived manner to the performed actions. With this information, it can adapt its behavioral routines to maximize the patient's state of mental wellbeing by, for example, avoiding those actions that have previously been unfavorably received by the patient, and repeating those actions that have been favorably received, considering various situational factors that may be found to partially account for those preferences when actions are repeated in different contexts.

Besides adapting the behavior of the ML algorithm, based on the human mental engagement with respect to respective operations and interactions in the real-life context, an aspect, or aspects of the real-life context itself may be directly adapted, such as but not limited to switching on or off, or otherwise changing the state of a particular device operating in the real-life context.

Returning to the example of the nursing robot above, if the robot has no information concerning the patient's preferences with respect to a certain action or state, the robot may actively decide to perform those actions for the specific reason of learning the patient's preferences, by adapting the context. For example, it may regulate the temperature or lighting conditions in the room for the sole purpose of evaluating the patient's implicit mental responses to the changed conditions, based on which responses the robot can learn the patient's preferences and work to adhere to these preferences in the future.

At least one of the analysis and inferencing by the machine learning algorithm and aspects of the real-life context may also be adapted by the information processing device based on engagement of the information processing device with respective operations and interactions in the real-life context.

For example, in case the ML algorithm misses information to complete a specific task or process at hand or is otherwise not able to associate identified data with sufficient quality and reliability, aspects of the context may be momentarily adapted to retrieve the required or missing information. That is, an aspect or aspects of the context may be adapted by introducing, changing, or deleting information, processes, tasks, events etc. to invoke a response of the participating human, either consciously or subconsciously.

Such adaptation may involve any of the momentary actions or processes handled, a momentary technological state of devices operating in the real-life context, but also virtual adaptations to invoke a particular mental response of the participating human to investigate a moral opinion, for example. In this way, like in humans, knowledge, proclivities, preferences, and moral values can be built up by the ML algorithm up on a trial-and-error basis to improve itself by means of Active Learning and cognitive/affective probing.

As the analysis and inferencing by the machine learning algorithm and the context itself, i.e. aspects thereof, can be controlled and adapted as a result of the data processing, the present method serves to impart human knowledge, intelligence, and subjective interpretations as well as human advise about tasks, processes, devices, and information perceived and the ML algorithm changing its own actions accordingly, in both training of and performing operations in real-life contexts.

According to a further aspect of the present method, the real-life context may comprise a specific cognitive probing event for training purposes.

Cognitive or affective probing refers to an application of active learning or active sampling where repeated sampling is done by eliciting implicit brain responses. A cognitive or affective probe is a technological state change that is initiated or manipulated by the same technology that uses it to learn from the user's implicit, situational, cognitive, or affective brain response to it. Cognitive probing or affective probing, then, is the repeated use of probes in order to generate a model in which the learned information is registered. The term cognitive probing is used as the general term including both cognitive and affective probing.

The method presented is not limited to the processing of human brain activity data of a single human individual but may also be practiced for the processing of brain activity data of a group of individuals participating in a respective real-life context. Referring to the example of autonomous vehicle driving above, the situational awareness of the road users may be identified from multiple occupants of the vehicle, if applicable, i.e. their mental engagement with the real-life context, and subsequently extract from the brain activity of respective or all occupants their moral alignment with respect to the road users.

Brain activity data may then be acquired from each individual of a group separately, while mental engagement with the context may by sensed of the group as a whole, of a sub-group or for each individual separately. This, dependent on a particular context, as will be appreciated.

By processing brain activity data of multiple persons involved, training of an ML algorithm can be significantly speeded up compared to training by a single user or can make use of a group consensus or a majority vote rather than individual judgements thus making the resulting information more reliable or more general, for example. In operation, differences in the aspect or aspects sensed from human mental engagement among the individuals of a group as well as differences in the evaluation and perception of operations and interactions among individuals of group participating in a common real-life context, may reveal additional operational information for controlling and/or adapting the ML algorithm and/or the context more quickly compared to an individual user, for example.

According to an aspect of the present method, human mental engagement with an aspect of the real-life context is identified from human bio-signal data sensed by at least one bio-signal sensor, including the pBCI.

Bio-signals, in the context of the present application, are signals that are generated by or from human beings, and that can be continuously measured and monitored by commercially available sensors and devices.

The term bio-signals refers to both electrical and non-electrical time-varying signals. Electrical skin conductivity, heart rate variability, body temperature, gestures, body movements, pupil dilation, eye movements, gaze, etc. are non-limiting examples of bio-signals. Brain waves, i.e. brain activity data are also bio-signals for the purpose of the present method. Which type of bio-signal sensor is to be used may be selected based on a particular context and/or a specific human participation, for example.

Like pBCI data, human physiological measurements, or bio-signal data for sensing human mental engagement of an individual or a group of individuals participating with a respective context can be acquired based on implicit human participation. In practice, besides the pBCI already used, in addition to or as an alternative for this pBCI, one or more separate bio-signal sensors may be used for sensing and assessing implicit human participation with the context, in accordance with the present method.

Referring again, by way of example, to the autonomous vehicle example above, the awareness of the occupant or occupants, alternatively or in addition to the pBCI output, in accordance with present application, can be based on data provided by human bio-signal sensors such as eye-tracking equipment worn by or otherwise operatively connected with or aimed at an occupant, or a head motion sensor, for example, in data communication with the information processing device operating the ML algorithm.

Other forms of prior art supervised Al use labels to learn to identify, localize, differentiate between, or recommend different kinds of objects or events. This requires that humans manually provide these labels by explicitly generating descriptors for concrete pieces of data such as images, videos, or audio. Implicit labelling using brain activity provides a method to do this faster for some types of descriptors, and may provide descriptors that may not be possible to generate in any other way.

An ML system arranged in accordance with the present methods, operating in a store may identify through body motion sensing or eye tracking which items visitors are looking at, and may identify by means of a pBCI their emotional or cognitive response upon viewing that item. Cognitive probes may additionally be used to specifically present the visitor with a range of items available in the store. The visitor's implicit responses may be reflected in patterns of brain activity that reflect for example subjective preferences or purchase intentions.

These pieces of information can be jointly processed, with the mental responses providing labels or descriptors for the items in the store, allowing the ML system to generate individualized profiles of the visitors' preferences, generic profiles to classify the items in the store, individualized recommendations of other items to visitors, general recommendations with respect to the items for the store managers, and personalized and/or general dynamic changes in the layout of the store to highlight specific items which the ML system has learned or predicts to be preferred by the current visitors, for example.

The present method provides a mechanism that assesses human objective an intuitive intelligence directly and automatically out of the brain, not requiring explicit actions or awareness from a human participating in a given real-life context. This not only significantly improves ease of use and comfort to participate in a real-life context by a human user, but also avoids cognitive overload or any distraction from the ongoing context and significantly speeds up training and operation compared to ML requiring explicit user actions.

The method according to the present application is excellently applicable for processing operational data, human mental brain activity data and human mental engagement data in real-time or quasi real-time, and in particular for the processing data pertaining to a time critical context.

The present method is versatile and applicable in a variety of real-life contexts from which operational data originate pertaining to technological states and technological state changes of a technical device or devices operating in a respective context, in particular a device or devices controlled by the information processing device operating the ML algorithm. Technological states comprise any of but not limited to device input states, device output states, device operational states, device game states, computer aided design states, computer simulated design states, computer peripheral device states, and computer-controlled machinery states and respective state changes. A technological state change in a context is any action undertaken by a piece of technology.

The term technology collectively refers to any and all (connected) technological elements in any potential situation or real-life context. With a technology's state being its specific current configuration, a technological state change is thus any change in configuration that the technology undergoes.

In the light of the present disclosure and the attached claims, the term 'operational data' may comprise any sensed, perceived, or otherwise identified objects, facts, quantities, properties, and other aspects and elements present in a real-life context. It is noted that operational data in the light of the present disclosure and the attached claims also refers to information relating to a real-life context as such, i.e. environmental information obtained from sources not directly controlled by the information processing device, such as a weather forecast, for example.

Operational data may comprise physical data and/or virtual data originating from the real-life context. The term virtual data refers to data available from a software program or software application operating in a respective context. That is, for acquiring this type of data no separate sensors, measurement equipment or other peripheral data recording equipment are required.

Non-limiting examples of real-life contexts or fields at which the present method can be applied to already solve existing problems or extend the capabilities of given systems are human-computer interaction, human-machine systems, human-robotic interaction, robotics, assistive technologies, medical technology, treatment/curing of health conditions, artificial intelligence, brain computer interfacing, cyber security and cyber technology, as well as in the sectors of law enforcement and interrogation, mind control, psychological modification and weapon systems, or combinations of such fields.

Reinforcement learning is an ML method that allows learning from trial and error. Each approach to solve a problem or each step in solving the problem is evaluated by a so-called evaluation or reward function. This function provides a score that indicates how well the approach or step worked in the solution. With that, a comparison is made with other approaches or steps and the best solution is identified or approximated over time.

Deep reinforcement learning uses methods from deep learning to learn from the evaluation function. Based on many iterations and large amounts of data (deep) reinforcement learning has provided solutions for problems.

However, for applications in real contexts, a problem occurs in finding an appropriate reward or evaluation function that can interpret a given context according to the solution step provided by an ML algorithm to be used for reinforcement learning.

In a yet further aspect of the method presented, the information processing device operates a reinforcement machine learning algorithm, in particular a deep reinforcement machine learning algorithm, wherein the implicit human mental brain activity data provides the reward function.

As mentioned above, one leading theory explaining how human intelligence is formed is the theory of predictive coding. The main concept is that the human brain constantly predicts the (near) future of its own state and the input it receives. After progressing to that timepoint, the real state of the human brain and its received input is compared to the initial prediction. With that, the prediction mechanism is evaluated and tuned to work better in future predictions. This approach is very similar to the above-mentioned reinforcement learning. The important part is that the brain generates 'its own evaluation function' by itself.

As the present patent application presents a mechanism that assesses human objective and intuitive intelligence directly and automatically out of the brain, not requiring explicit actions from a person participating in a given context, it is the insight of the inventors to use the implicit human mental brain activity data processed in accordance with the present method as the reward or evaluation function of a reinforcement ML algorithm. When a human is in the same context as an ML algorithm to be trained, the human's implicit brain responses may serve as an evaluation function for that ML algorithm.

Because the assessed human mental brain activity data can not only be interpreted binary, i.e. correct or wrong, but also continuously, i.e. any number between 0 and 1, indicating the degree of subjective agreement to the perceived contextual event, the method presently presented may be used to support an Artificial General Intelligence, AGI, data processing algorithm for handling plural tasks in real-life contexts with intelligence proportional to human general intelligence.

For example, a machine working in the same context as a human user can thus use the human reward or evaluation function as a context-sensitive evaluation function for its own (deep) reinforcement learning. Other mental states, such as agreement or satisfaction, can also be seen as an implicit judgement of perceived information, and may be utilized in a similar function.

This approach can be defined as Neuroadaptive (Reinforcement) Learning - a method that allows to generate Artificial Intelligence, Al, that learns to solve problems based on the interpretation/evaluation of a certain human. As used herein, neuroadaptive reinforcement learning refers to an ML algorithm that uses human mental brain activity data to model how an agent should take actions in an environment (real-life context) to further a goal or maximize a reward. In the light of the present disclosure and the attached claims, the term 'human mental brain activity data' may refer to any sensed, recorded, or otherwise obtained data, be it raw, processed, classified, labelled, converted, transformed, or augmented, originating from and reflecting the activity of a human nervous system.

Hence, the current method provides a tool to automatically assess a human brain's implicit interpretation of a perceived event in a given real-life context, in quasi real-time after the occurrence of that event. Such a tool provides for both continuous and event-related monitoring of the mental states of the human and allows an automized view in the subjective, situational interpretation of a person and allows to make this information available for further processing, such as to transfer key aspects of the cognition and mindset of a human into a machine.

Building up on this, it is found that through neuroadaptive reinforcement learning AI can converge to particular aspects of the human mindset, reflecting this person's intelligence and moral values. And the more data that become available from a person over time and in different contexts, the more history can be built-up, and a better match to that one person's intuitive intelligence is established, building up a profile/model of that human's subjective interpretations, that can be referred to as a cognitive copy.

Neuroadaptive learning in accordance with the present method is, in a broader sense, not limited to reinforcement learning, but may be applied with other ML algorithms, such as but not limited to a multi-modal network with supervised learning, Monte Carlo techniques, Temporal Difference learning, and Q learning.

A practical implementation of the present data processing method, implemented in an information processing device operating a machine learning algorithm for processing operational data originating from a real-life context and human mental brain activity data relating to implicit human participation with such context, may comprise the steps of:
- identifying, by the information processing device, an aspect of the real-life context, the aspect identified from sensing human mental engagement with the real-life context;
- acquiring, by the information processing device, operational data pertaining to the identified aspect;
- acquiring, by the information processing device, human mental brain activity data of implicit human participation with the real-life context pertaining to the identified aspect;
- processing, by the machine learning algorithm, the acquired operational data and mental state data assessed from the acquired human mental brain activity data, and
- controlling, by the information processing device, at least one of analysis and inferencing by the machine learning algorithm and aspects of the real-life context based on the data processing.

Those skilled in the art will appreciate that, the information processing device may operate multiple algorithms, such as but not limited to at least one further algorithm for at least one of sensing, performing, acquiring, processing, and pre-processing of at least one of brain activity data, operational data, human mental engagement, aspect identification, mental state data, human bio-signal data and for control or adaptation of the machine learning algorithm and aspects of the context. The controlling step may comprise associating the identified operational data and human mental brain activity data.

Besides a single ML algorithm, parts of the processing of data according to the present method may be performed by plural ML algorithms operated by a single or multiple cooperating information processing devices, or so-called virtual machines, which processing is deemed to be covered by the scope of the attached claims.

Following the method presented, in case the information processing device operates multiple algorithms as indicated above, potentially all such algorithms may be adapted based on the data processing performed, inclusive multiple ML algorithms and data storage facilities.

In a second aspect the present application provides a program product, comprising, i.e. encoded with instructions stored on any of a transitory and a non-transitory medium readable by an information processing device, which instructions arranged or configured to perform the method according to any of the aspects disclosed above when these instructions are executed by an information processing device, including any of a computer and a computer application.

In a third aspect the present application provides a data processing system, comprising means arranged for performing the data processing method disclosed in conjunction with the first aspect above.

In general such a processing system includes at least one information processing device arranged for operating or running a machine learning algorithm, equipment in data communication with the information processing device for acquiring operational data originating from a real-life context, and at least one passive brain-computer interface in data communication with the information processing device for acquiring human mental brain activity data of a human participating in that context, and at least one bio-signal sensor in data communication with the information processing device for sensing human mental engagement with an aspect of the context. As explained above, the at least one bio-signal sensor may be provided by the at least one passive brain-computer interface.

An embodiment of the present invention provides a system comprising a processor, memory, a context sensor, and a scalp electroencephalogram, wherein the processor runs a machine learning algorithm from the memory comprising processing operational data originating from a real-life context from the context sensor and human mental brain activity data relating to implicit human participation with this real-life context and provided by the scalp electroencephalogram.

In a fourth aspect, the present application comprises a trained machine-learning model trained in accordance with the data processing method disclosed in conjunction with the first aspect above. The machine learning algorithm may be trained 'on the fly', i.e. in a real-life context, or based on training data comprising known real-life context data, and human mental brain activity data relating to implicit human participation with the (known) real-life context.

The present method, system, program product and trained ML model provided results in what is herein called a Situationally Aware Mental Assessment for Neuroadaptive Artificial Intelligence, SAMANAI, tool that enables an ML algorithm to learn from a human brain directly in real-life contexts, environments or scenarios comprising multiple sources of information, processes, tasks, events, etc.

This SAMANAI tool can be applied for increasing the learning rate of an ML algorithm by integrating preprocessed evaluation of the human mind into the analyzing and inferencing procedure of the ML algorithm, without the need of active input by the human participant or observer, i.e. implicitly, with the effect that the learning will be quicker and less erroneous than using human input through button presses.

The SAMANAI tool can be applied to personalize any ML algorithm, either a strong or weak Al, by assessing subjective, human values provided in a given real-life context environment and creating a model of these values into the ML algorithm for future decision making and inference. A moral agent may be created that is based on a moral model inferred from a human or a group of humans.

The SAMANAI tool enables context dependent valuations, interpretations, assertions, labelling, etc. by the ML algorithm of operational data in real-life contexts or environments, handling multiple, versatile, and non-predefined tasks.

As an untrained ML algorithm is incapable of evaluating the real-world on its own, by definition, SAMANAI provides information to the learning process that would not be available otherwise.

The above-mentioned and other aspects of the present application are further illustrated in detail by means of the figures of the enclosed drawings.

### Brief Description of the Figures

Figure 1 illustrates, schematically, an example of a general set-up for practicing embodiments of the present invention in a real-life operational application and for training purposes.
Figure 2 shows an example of electrode placing for a passive brain-computer interface for registering brain activity signals based on electroencephalography, that can be used for operating the teachings of the present invention.
Figures 3a and 3c illustrate measured event-related potentials and Figures 3b and 3d display scalp projections of the human brain activity regions involved, in an example of the present invention.
Figure 4a illustrates measured event-related potentials and Figure 4b displays human brain activity regions involved, in another example of the present invention.
Figures 5a and 5c illustrate scalp patterns produced and Figures 5b and 5d show filtered brain activity, in a further example of the present invention.
Figure 6 illustrates, in a flow-chart type diagram, an example of steps of the method according to an embodiment of the present invention.

### Detailed Description

The present application relates to data processing performed by and implemented in an information processing device operating a machine learning algorithm arranged for processing a plurality of operational data acquired from devices operating in a certain context and human mental brain activity data of human participation with or in that context, provided by a passive brain-computer interface.

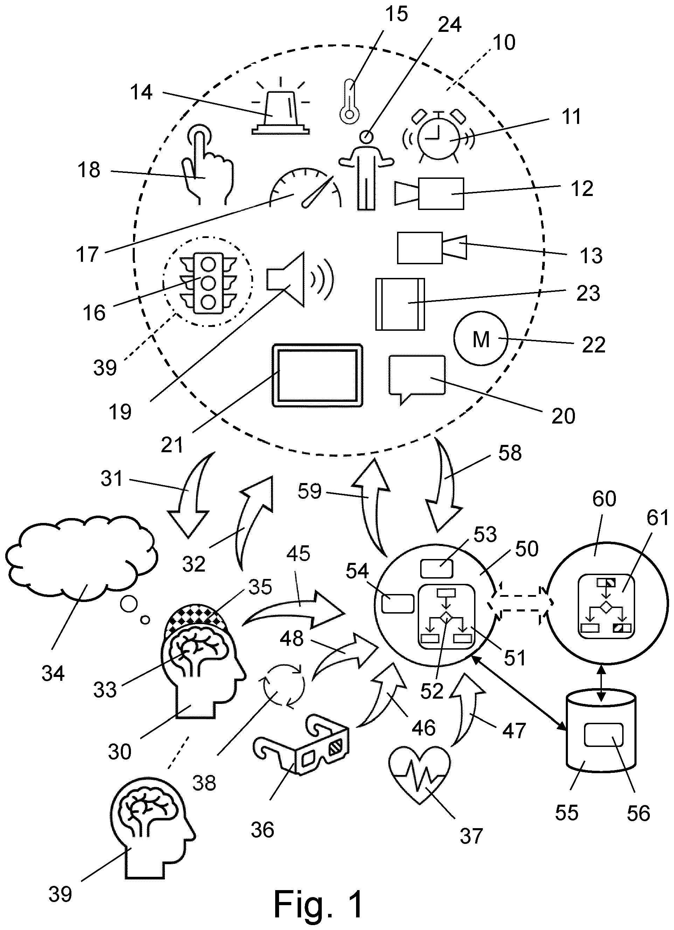

In Figure 1, reference numeral 10 schematically represents a particular real-life or real-world context, that is a non-predefined or authentic context or environment, occurring in reality or practice, illustrated by a dashed line, typically composed of a plurality of devices performing and handling multiple, different, and non-predefined tasks.

Non-limiting examples of such devices are audible alarms, illustrated by an alarm clock 11, sensing camera's such as a daylight camera 12 and/or a night vision Infra-Red, IR, camera 13, lighting devices 14, including flashing lights, temperature sensors or meters 15, traffic lights 16 and speedometers 17 in case of a driving vehicle context, for example, (virtual) buttons, keys or knobs 18 to be operated, audio equipment 19, message systems 20 presenting spoken, written or video messages, a display or displays 21, and one or more actuators represented by a motor 22.

Reference numeral 23 represents a software application, for example control software, simulation software, gaming software, communication software, or so-called apps.

Reference numeral 24 refers to at least one organism or living being, such as a person or persons, or an animal or animals acting in the context 10. In the light of the present application, the organism or living being forms part of the context 10 and acts performed by, and behavior observed from, such an organism or living being are treated as operational data originating from the context 10.

It will be appreciated that in practice a certain context may be comprised of more or less of these devices or other devices performing other tasks or processes, such as described in the Summary part above, for example.

Reference numeral 30 refers to a human being participating in or with the context 10. The manner and degree of participation or involvement of the human 30 may vary dependent on a respective context. In some scenarios the human 30 will only perceive actions and events occurring in a context 10 and has no control over the devices, for example, while in other scenarios the human is also actively involved in the operations occurring in the context 10, such as pushing buttons 18, turning on lights 14, evoking alarms 11, etc.

Curved arrows 31, 32 schematically represent the human participation. Arrow 31 illustrates the perception of the context 10 by the human 30 and arrow 32 illustrates active interaction of the human 30 with the context 10, i.e. the human 30 is in charge with and/or controls tasks, processes and operations that occur in the context 10 and/or need to be accomplished.

When the human 30 is not particularly focused on events, changes, adaptations, modifications etc. that happen in or in relation with the context 10 they nevertheless may attract attention, arousal or are may otherwise be noticed 31 by the human 10, consciously or subconsciously.

Reference numeral 50 refers to an information processing device, typically including a computer, a computing device, a virtual machine, a server, or a plurality of cooperatively operating computers, computing devices, virtual machines, or servers, either operating stand-alone or on-site and/or in a cloud computing environment (not specifically illustrated).

The information processing device 50 operates a Machine Learning, ML, algorithm 51 arranged for processing a plurality of operational or contextual data acquired from the devices 11 - 23 operating in the context 10. The ML algorithm 51 is typically arranged for analyzing and inferencing data processed by the ML algorithm 51, as schematically indicated by reference numeral 52.

Besides a single ML algorithm 51, parts of the processing of data according to the present method may be performed by plural ML algorithms operated by a single or multiple cooperating information processing devices 50.

Acquisition of operational data from the context 10 is illustrated by a curved arrow 58. Operational data for controlling, by the information processing device 50, of one or more of the devices operating in the context 10 is illustrated by a curved arrow 59.

Those skilled in the art will appreciate that the devices 11 - 23, when operating in the context 10, are in data communication with the information processing device 50, either via an individual or shared wired, wireless or cloud or internet data communication connection, for example operating an Internet-of-Things, loT, a WiFi, a Bluetooth^{™} or any other data communication protocol.

Brain activity signals 33 of the human 30 participating with or in the context 10 are provided by a passive Brain-Computer Interface, pBCI. In the embodiment shown, the pBCI is illustrated in two parts that are in data communication with each other, illustrated by a curved arrow 45 representing brain activity data of the human 30.

That is, a hardware part 35 for registering brain activity signals 33 of the human 30, such as a plurality of electrodes attached to the human head in the case of an electroencephalogram, EEG, and a software part 54 running on the information processing device 50, for processing the raw brain activity signals received from the hardware part 35 of the pBCI. Commercially available pBCI software 54 for use with the present method includes BCILAB and OpenViBE, for example. In practice, prior to registering brain activity data by a pBCI, a calibration procedure may have to be applied.

For the purpose of the present application, the software part 54 may process multiple pBCls each operating a different classifier directed to sense different brain activity associated with different types of response or mental states of a human participant may be used. In practice, tens or hundreds of classifiers may be deployed.

Electroencephalography is a well-known electrophysiological monitoring method to record electrical activity on the scalp representing the activity of the surface layer of the brain underneath. It is typically non-invasive, with the electrodes placed along the scalp. Electrocorticography, involving invasive electrodes, is sometimes called intracranial EEG. Both methods are applicable with the present method.

The ML algorithm 51 may comprise a reinforcement machine learning algorithm, in particular a deep reinforcement machine learning algorithm, comprising a reward function, wherein the implicit human mental brain activity data of the pBCI 35, 54 serve as the reward function.

ML software that may serve as a basis for operating the present method is available as open-source software, such as TensorFlow, Keras, and scikit-learn, for example.

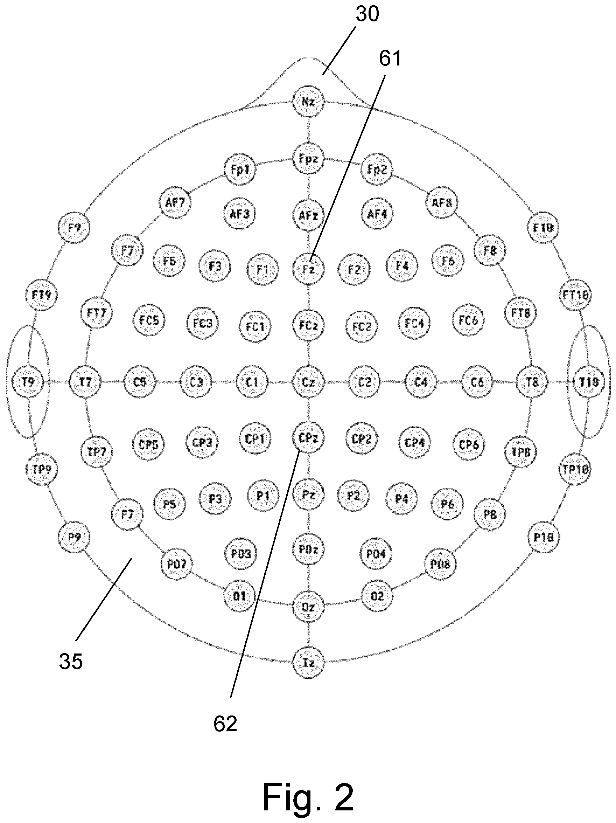

Figure 2 shows a typical electrode placing of the hardware part 35 of a pBCI for registering brain activity signals based on EEG and used for operating the teachings of the present invention.

Instead of or in addition to recording brain activity data 45 by EEG or intracranial EEG, other recording techniques suitable for the purpose of the present invention are Magnetoencephalography, MEG, a functional neuroimaging technique for mapping brain activity by recording magnetic fields produced by electrical currents occurring naturally in the brain, functional Near-InfraRed Spectroscopy, fNIRS, that is a functional neuroimaging technique based on brain hemodynamics, functional Magnetic Resonance Imaging, fMRI, that measures brain activity by detecting changes associated with blood flow, or ElectroCorticoGraphy, ECoG, a type of electrophysiological monitoring that uses electrodes placed directly on an exposed surface of the brain, i.e. not on the scalp, to record electrical brain activity.

By the above-mentioned techniques, pBCI data are provided based on implicit or passive human participation, different from explicit or active, i.e. voluntary, intentional, conscious, human interaction with the context 10.

Those skilled in the art will appreciate that future devices based on any of these techniques or future new techniques may be used for providing brain activity data for the purpose of the present invention.

A dash-dotted circle in Figure 1 schematically illustrates an aspect 39 of the context 10 with which the human 30 is momentarily in mental engagement, in accordance with the present method, sensed from human physiological measurements. For example, it is sensed that the attention of the human 30 is attracted by a change in the traffic light 39, for example from green to orange, in a context of an occupant of an autonomously driving vehicle. As the context 10 changes continuously, at a later moment in time the human 30 may be engaged with another aspect, such as alarm 11, etc. Hence, over time, the human is mentally engaged with a plurality of aspects 39 of the context 10.

Measurements of the physiological state of the human 30 can be continuously monitored from the brain activity signals 33, for example by the pBCI 35 or a separate pBCI (not shown), as well as from measurement data provided by sensors and devices attached or operatively connected to the human 30.

Sensors for human physiological measurements such as measurements of bio-signals for use with the present method are commercially available. Examples of such sensors are eye-tracking equipment 36, heart rate monitoring equipment 37 or body motion sensors 38, that are worn or otherwise operatively connected with the human 30 and are in data communication with the information processing device 50, as illustrated by curved arrows 46, 47 and 48, respectively.

Which type of bio-signal sensor is to be used may depend on a particular context and/or a specific human participation, for example. It will be appreciated that the bio-signal sensors shown in Figure 1 and discussed above are just examples of suitable sensors and any other or future bio-signal sensor may be applied with the teachings of the present invention, such as but not limited to direct and indirect measurements of electro cardiac activity, body temperature, eye movements, pupillometric, hemodynamic, electromyographic, electrodermal, oculomotor, respiratory, salivary, gastrointestinal, and genital activity.

It is noted that the human bio-signal data communicated 46, 47, 48 to the information processing device 50 may also be processed by one or a plurality of classifiers arranged for processing the bio-signal data 46, 47, 48 received from the bio-signal sensors 36, 37, 38, and also using commercially available software operated by the information processing device 50.

Those skilled in the art will appreciate that the information processing device 50 may operate plural ML algorithms 51 and/or plural algorithms for data sensing of sensors 36, 37, 38, input/output of operational data 58, 59, processing and pre-processing in real-time or quasi real-time, and time-synchronization, in particular in a time critical context, of at least one of brain activity data, operational data, human mental engagement data, aspect identification data, mental state data, bio-signal data, and for control and adaptation of the machine learning algorithm 51, i.e. the analyzing and inferencing part 52 thereof. As well as for controlling and adapting aspects of the context 10, such as controlling the alarms 11, lighting 14, audio equipment 19, actuator(s) 22, etc. or purposely inducing probes to evoke a response from the human 30. Such algorithms are collectively referred to by refence numeral 53.

For completeness's sake, reference numeral 55 represents a database or data repository or any other type of memory devices for storing data acquired and processed by the information processing device 50, such as is generally known. The database may be fully or partly located externally from the information processing device 50, for example remote in a cloud computing environment or data center, and/or be fully or partly located internally with the information processing device 50.

The database 55 may contain files, lists, libraries, or any other collection of information for use in the processing of data according to the present invention, as generally designated by block 56. As disclosed in the Summary part above, the more data that become available from a person over time and in different contexts, the more history can build up a profile/model of that human's subjective interpretations, that can be referred to as a cognitive copy, which may be stored in the database 55.

When the information processing device 50 operates multiple algorithms 52, 53 as described above, potentially all such algorithms may be controlled and adapted based on the data processing performed in accordance with the present method, inclusive control and adaptation of multiple operated ML algorithms 52, data storage 55 and respective contexts.

Operational data 58, 59 may comprise physical data produced by or for the control of physical devices, equipment, sensors, etc. in the context 10, such as technological device states like device input states, device output states, device operational states, device game states, computer aided design states, computer simulated design states, computer peripheral device states, and computer-controlled machinery states and respective state changes, as well as so-called virtual operational data originating from the context 10. The latter are data relating to, for example, the software program or software application 23 operating in the context 10, and/or data received from the camera's 12, 13, for example, or any other sensor operating in the context 10.

Referring to the Summary part above, the embodiments of the present invention provide for a continuous and event-related monitoring of the human mindset and mental states of the human and allows an automatic view into the knowledge, intelligence, moral values, subjective and situational interpretations as well as human advise about tasks, processes, devices, and information perceived by the human. In Figure 1, the human mindset and mental states is very schematically illustrated by the brain cloud 34.

The set-up discussed above is representative for both a real-life operational application and as a cognitive probing context 10.

Although a single human 30 is referred to in Figure 1, those skilled in the art will appreciate that the set-up presented is feasible for more than one human participating in or with the context 10, such as a group of people, by simply replicating the pBCI 35, 54 and the sensors 36, 37, 38, and using processing software adapted accordingly, for example. Schematically indicated by the further human 49.

Further, the set-up presented is ideally suited for operating an Artificial General Intelligence, AGI, data processing ML algorithm.

The present invention also comprises a program product comprising instructions stored on any of a transitory and a non-transitory medium 60 readable and executable by an information processing device 50.

The embodiments according to the present invention also provide a trained machine-learning algorithm or model 61, that enables an ML algorithm to learn from a human brain directly in contexts, environments or scenarios comprising multiple sources of information, processes, tasks, events, etc. The trained model 61 may be stored on any of a transitory and a non-transitory medium 60 readable and executable by an information processing device 50, as illustratively shown in Figure 1.

As discussed in the Summary part above, the present method, system, and trained ML model provided results in a what is called a Situationally Aware Mental Assessment for Neuroadaptive Artificial Intelligence, SAMANAI, tool that enables an ML algorithm to learn from a human brain directly in real-life contexts, environments or scenarios comprising multiple sources of information, processes, tasks, events, etc. The SAMANAI tool may take the form of a program product, comprising instructions stored on any of a transitory and a non-transitory medium 60 readable and executable by an information processing device 50.

Returning, by way of example, to the autonomous driving vehicle scenario disclosed in the Summary part above. The set-up shown in Figure 1 is partly applicable to this example.

Ideally, the ML algorithm operating the vehicle would have access to the general moral alignment of its occupant, as well as any momentary moral judgements to inform any immediate decision to be made. This can be achieved by monitoring and registering brain activity related to moral interpretations of ongoing situations during driving. To that end, a SAMANAI-enabled autonomous vehicle may contain the following elements and may function as subsequently described.

The SAMANAI-enabled autonomous vehicle is or can be fully operated by its ML system. This ML system has visible-light and infra-red cameras, radar, and lidar sensors on the basis of which it is capable of identifying specific objects and events in the external environment using state of the art object localization and recognition algorithms such as region-based convolutional neural networks. Furthermore, this ML system contains a software model that enables it to make decisions in morally critical situations, such as situations where a collision avoidance decision has to be made. Furthermore, this Al has access to a list of situations, events, and objects that have been identified by human experts as having a moral dimension: this list contains objects such as children, seniors, non-autonomous vehicles, cyclists, etc. and may be stored in the database 55 as a file 56, for example.

The autonomous vehicle also possesses internal sensors with which the Al can monitor and identify the behavior of the vehicle's human occupant 30. A camera 12 and motion sensor 38 is used to monitor the occupant's head position and orientation, and an eye tracking system 36 is used to monitor their eye gaze and blinking behavior. Furthermore, the vehicle is equipped with an EEG system 35 to monitor the occupant's brain activity 33. The system contains thirty-two electrodes, such as those shown by small circles in Figure 2, worn by the occupant 30 providing broad coverage of the occupant's scalp, twelve of which in particular are placed such that they cover the projection sites and the projection boundaries of the occipital-parietal areas of the cerebral cortex and the precuneus, which electrodes are connected wirelessly through Bluetooth to an amplifier sampling the occupant's brain activity at 100 Hz in order to accurately capture spatiotemporal variations of at least 50 ms in duration.

It is noted that in practice, the electrodes shown in Figure 2 may be differently arranged.

The vehicle also has all necessary software algorithms 53 to process the internal sensors and extract from them the mentioned aspects of the occupant's head position and location and gaze direction. For the brain activity in particular, the vehicle possesses a preprocessing algorithm which is capable of state of the art artefact rejection, including Artefact Subspace Reconstruction, ASR, and Online Recursive Independent Component Analysis, ORICA, see, e.g. S. -H. Hsu, T. R. Mullen, T. -P. Jung and G. Cauwenberghs, "Real-Time Adaptive EEG Source Separation Using Online Recursive Independent Component Analysis," in IEEE Transactions on Neural Systems and Rehabilitation Engineering, vol. 24, no. 3, pp. 309-319, March 2016, and capable of band-pass filtering the data, in particular between frequencies of 1 and 15 Hz and 1 and 30 Hz in the spectral domain, as well as other common digital signal processing operations such as re-referencing, channel rejection, epoching, interpolation, and subsampling.

As used herein, ASR refers to an adaptive method for the online or offline correction of artifacts comprising multichannel EEG recordings, see, e.g. T. R. Mullen, C. A. E. Kothe, Y. M. Chi, A. Ojeda, T. Kerth, S. Makeig, T. -P. Jung, and G. Cauwenberghs, "Real-Time Neuroimaging and Cognitive Monitoring Using Wearable Dry EEG," in IEEE Transactions on Biomedical Engineering, vol. 62, no. 11, pp. 2553-2567, November 2015.

The other internal and external sensors operate at least 100 Hz in order to retain synchrony with the EEG. Different sensors may operate at different frequencies, which can be handled by proper synchronization software, for example, as mentioned above, or any other of the software 53 executed by the information processing device.

The autonomous vehicle furthermore contains two passive brain-computer interfaces, pBCls, implemented as software algorithms 54 with access to above-mentioned EEG system's measurements of the occupant's brain activity 33. One pBCI is capable of identifying visual processing of perceived objects and events. The other pBCI is capable of identifying moral evaluations of perceived objects and events. These are both implemented using state of the art feature extraction techniques, in particular the extraction of the mean amplitude values on all or selected channels in any number, for example eight, consecutive non-overlapping windows following identified events, and using state of the art machine learning applications to differentiate between classes of events, in particular using linear discriminant analysis with feature dimension regularization.

These two pBCI algorithms are capable of producing, from measured brain activity plus an onset marker indicating a specific event, a binary estimate of the visual processing of the respective event, and a binary estimate of the moral judgement of the respective event, as well as an estimate of the accuracy of these estimates, in real time or quasi-real time. It will be appreciated that the vehicle may comprise more than two pBCls.

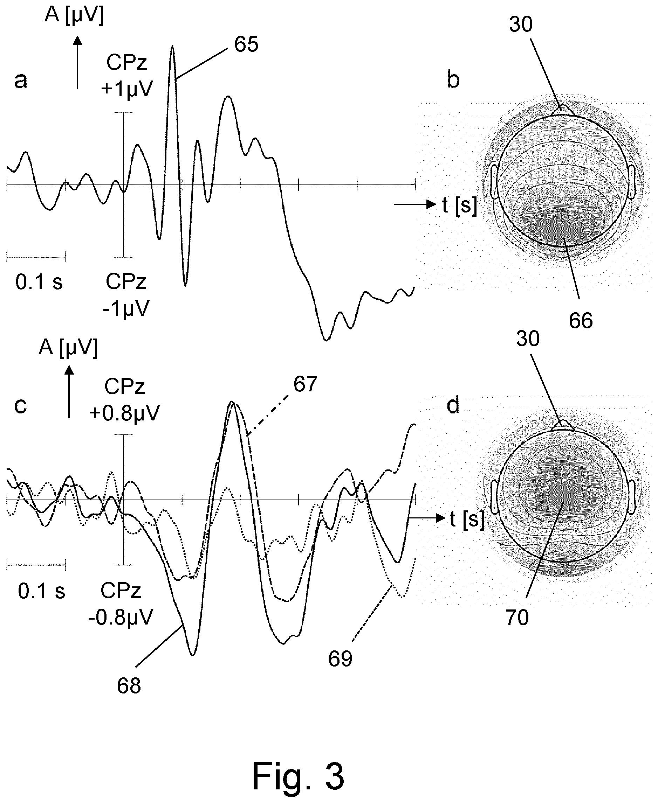

Sample brain activity reflecting visual processing and moral evaluation, which the above-mentioned pBCls will be capable of identifying as such, are presented in Figure 3.

The autonomous vehicle furthermore contains all the hard- and software components necessary to bundle and simultaneously record 55 the data generated by all above-mentioned sensors, in particular a software implementation, such as one similar to the ANSI/CTA-2060 standard providing a streaming and storage format combining continuous time series and static metadata of multiple modalities potentially operating at different frequencies. With these described capabilities, a sample scenario of SAMANAI activity is as follows.

The Al is steering the autonomous vehicle along a path while its external sensors continuously monitor the objects and events in the external environment. While doing so, it detects an object in its environment that is contained in its list 56 of objects with moral relevance. Upon identifying this object, it ascertains whether or not the occupant 30 is likely to have perceived the same.

To that end, it identifies whether or not the occupant's head location, position, and eye gaze are such that the object may have been perceived. Simultaneously, it identifies whether or not the visual processing pBCI has detected visual processing. If the combined information from these two processes provides an above-threshold likelihood that the occupant perceived and processed the object, the moral evaluation pBCI is utilized to identify the occupant's moral evaluation of the apparently perceived object.

If this pBCI produces an above-threshold likelihood of successful identification of this moral evaluation, which is either negative or positive (adverse or favorable), this information is fed back to the ML algorithm. The ML algorithm then uses this additional information to adjust its decision-making model in morally critical situations, such that this model more closely matches the moral judgements of the occupant 30.

To this end, the ML algorithm uses its inherent inference and analysis methods 52 on the basis of all previously and newly available information considering their estimated accuracy.

The SAMANAI tool 61 thus enables the autonomous vehicle to adjust the ML's decision process for morally critical scenarios without at any point requiring any explicit communication from the occupant to the vehicle.

Figure 3a illustrates graphically an average Event-Related Potential, ERP, 65 recorded by the inventors at electrode CPz 62, see Figure 2, following a change in the visual field of a human observer 30. Raw EEG data was referenced to electrode Fz 61, see Figure 2, filtered between 1 and 30 Hz, epoched relative to the change in the visual field, baseline-corrected, and averaged. Generally known ERP components related to visual processing such as the N70, P100, and N135 can be identified.

Figure 3b illustrates an interpolated scalp projection of brain activity originating from the occipital-parietal region 66 of the cerebral cortex where the visual processing represented by the ERP shown in Figure 3a primarily takes place.

Figure 3c shows an average event-related potential recorded by the inventors at electrode CPz 62, following the visual display of either morally negative, curve 68, or morally positive, curve 67, material, as well as the difference between the two ERPs, curve 69. Raw EEG data was referenced to the common average, filtered between 1 and 15 Hz, epoched relative to the onset of the respective visual material, baseline-corrected, and averaged.

Figure 3d shows an interpolated scalp projection of brain activity originating from the precuneus 70, where the moral processing represented by the ERP shown in Figure 3c primarily takes place.

In Figures 3a and 3c amplitudes are depicted along a vertical microvolt, µV, scale, running along a horizontal time scale t, where time is depicted in seconds, s.

Returning, by way of example, to the nursing robot example disclosed in the Summary part above. Again, the set-up shown in Figure 1 is partly applicable to this example.

The robot, tasked to support the patients as best it can, is operating in a highly complex environment where many of its own operational goals depend on the general or momentary preferences of a human patient 30. The SAMANAI tool 61 can be used to access these preferences and alter behaviors accordingly.

To this end, the SAMANAI-enabled robot would be equipped with hardware similar to that mentioned in the autonomous car driving example above, allowing it to sense both the environment and the patient. Furthermore, the ML system controlling the robot has a repertoire of behaviors, and, for each patient 30, a patient model or user model, in which the preferences of the respective patient can be stored. Both models may be stored as a file 56 in the database 55, for example.

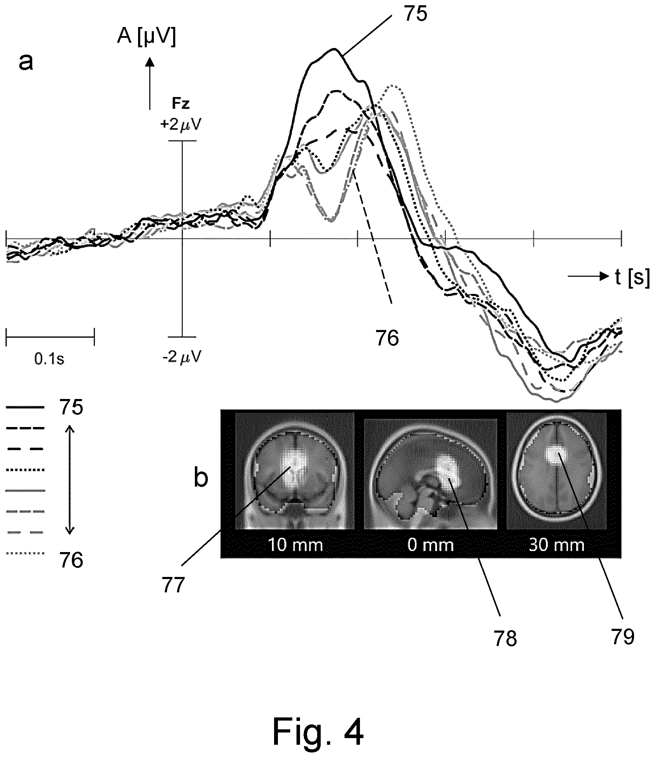

At least one pBCI algorithm 54 is present, capable of assessing robot actions as wanted or unwanted. The corresponding processed brain activity could, for example, look like the activity and originate in the cortical areas indicated in Figures 4a, 4b, 4c and 4d.

The ML system furthermore has access to additional physical and virtual sensors, like the ones as shown in Figure 1, providing situational parameters such as the presence and potentially the identity of other persons 49 (see Figure 1) in the environment, the time of day, the time since a last meal/medication/bathroom visit, the temperature, humidity, etc.

When the nursing robot decides to perform a specific action, the human patient 30 may interpret this in various ways: for example, they may or may not appreciate it, feel it is in error, or feel guilty for not having performed the task themself. Thus, whenever the robot performs or begins to perform a specific task, it ensures that the patient is aware of its actions by monitoring the patient's mental engagement with itself or the object of its actions.

This is done by at least one of tracking 36 the patient's eye gaze towards the action, identify the patient's attention-directing movements 38 towards the action through motion tracking happening at the same time the robot begins the task, sensing an auditory reaction from the patient related to the action, the intentional generation of noise or other events such as to ensure the patient's awareness, and evaluation of patterns of brain activity 33 dependent on the events taking place.

When, based on these measures, sufficient certainty is established that the patient is aware of the robot's actions, the patient's interpretation of those actions is implicitly derived from the patient's brain activity 33 using the passive brain-computer interface 35, 54. That is, the onset of the patient's mental engagement 16 is established and, relative to this onset, the patient's brain activity 33 is analyzed to produce an estimate of the patient's favorable or unfavorable interpretation of the respective action. Based on this, the robot ML algorithm can learn that, given the current context 10, as expressed through the sensed situational or operational parameters, the patient mentally responds in the perceived manner to the performed actions.

With this information, the SAMANAI-enabled robot can, in that very moment, change its behavior to reflect the patient's momentary preferences, as well as control and/or adapt its behavioral routines to maximize the patient's state of mental wellbeing in the long term by, for example, avoiding those actions that have previously been unfavorably received by the patient, and repeating those actions that have been favorably received, taking into account the situational factors that may be found to partially account for those preferences when actions are repeated in different contexts.

Furthermore, if the robot has no information concerning the patient's preferences with respect to a certain action or state, the robot may actively decide to perform those actions for the specific reason of learning the patient's preferences.

Figure 4a shows average event-related potential recorded by the inventors at electrode Fz 61, see Figure 2, following specific actions taken by an autonomous agent which the human observer 30 approves to varying degrees, separated into eight categories ranging from complete approval, solid black line 75, to complete disapproval, dotted grey line 76. Raw EEG data was referenced to the common average, filtered between 0.1 and 15 Hz, epoched relative to the visible onset of the action taken, baseline-corrected, and averaged. The amplitude between approximately 150 and 200 ms scales linearly as a function of the degree of approval.

Figure 4b shows brain areas 77, 78, 79, i.e. the white spots in Figure 4b, identified by the inventors primarily contributing to the effect represented by the ERP shown in Figure 4a, highlighted on an average brain model. Brighter areas contribute more to the effect than darker areas.

When a human is in the same context as the ML algorithm, the human's implicit brain responses can serve as an evaluation function for that ML. As an example, a given ML system operating on a battlefield has a large number of sensors to perceive its environment: cameras, thermal imagers, microphones, radar, lidar, chemical composition sensors, seismometers, gyroscopes, et cetera. It is thus capable of recording this context and the events taking place within it to any possible degree of objective accuracy. With the appropriate algorithms, such as those mentioned in the above example of the autonomously driving car, it is also capable of identifying specific objects and events in that context.

A human observer in this same environment will be able to interpret it in a way that uniquely reflects their subjective, personal expertise and intuition. This human will pay closer attention to some aspects of the context than to others. SAMANAI-enabled, the ML algorithm in this context additionally has a brain monitoring system such as an EEG, MEG, ECoG or fNIRS system, with accompanying components including i.e. electrodes, an amplifier, and necessary signal processing algorithms. It will also have at least one pBCI capable of interpreting this brain activity as reflecting relevant judgements of the context such as surprise.

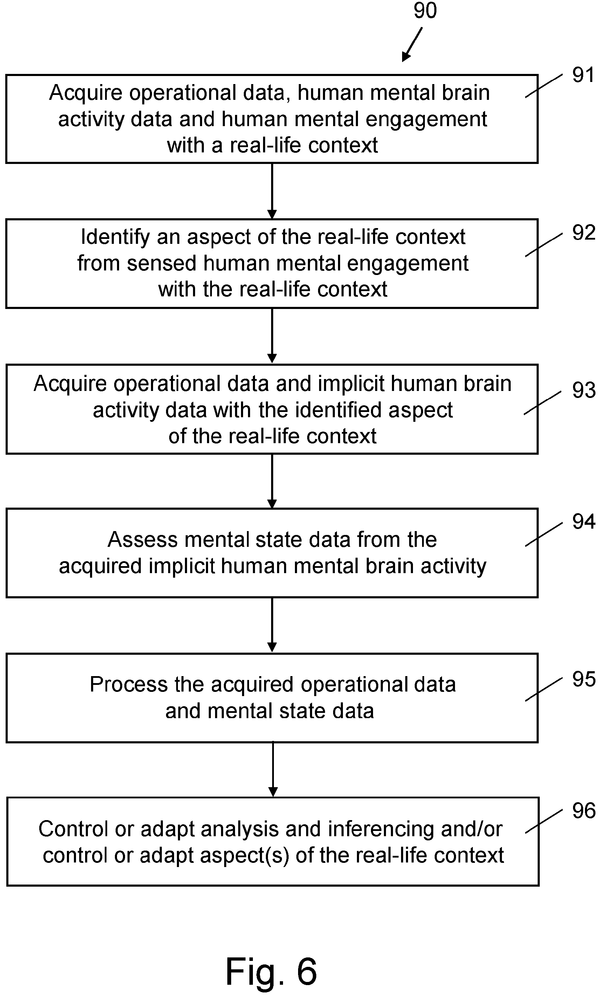

This pBCI can operate in an event-related fashion as described in the example of the autonomously driving car above, or can operate continuously using, instead of the feature extraction and machine learning methods mentioned in that example, the method of filter bank common spatial patterns following appropriate corresponding preprocessing steps, as illustrated with Figure 6.

Operating in an event-related fashion, the ML algorithm can track which parts of the context the human is attending to using human bio-signal data, e.g. its eye trackers and motion sensors. This human may, for example, observe a configuration in the environment reminiscent of a trap they have experienced before. This will automatically and potentially subconsciously evoke a pattern of brain activity that signals the significance of this aspect of the context. The pBCI, operating at least one classifier, can detect and classify this response and provide it to the ML, essentially serving as an additional human sensor providing an evaluation of aspects of the environment. The Al, now having available to it a representation of the real-life context, a representation of a specific aspect of this context identified through the human's engagement with it, and the human's mental evaluation of this aspect, can jointly and/or separately process these pieces of information using its inherent inference and analysis methods in order to control or learn how to respond or adapt to different aspects of the context.

Operating in a continuous fashion, the ML algorithm may be exhibiting behavior of a continuous nature, such as moving towards the edge of a building. Distance to this edge is a continuous variable with dynamically associated risk. Throughout the duration of this behavior, the Al can assess the human's mental engagement with this behavior as stated before, and continuously (following every n newly recorded samples, or with a given frequency) request from the continuous pBCI an evaluation of the current mental state of the human with respect to their approval of the current situation. This continuous interpretation is then jointly processed with operational data, allowing the Al to control or learn the appropriate behavior in this situation.

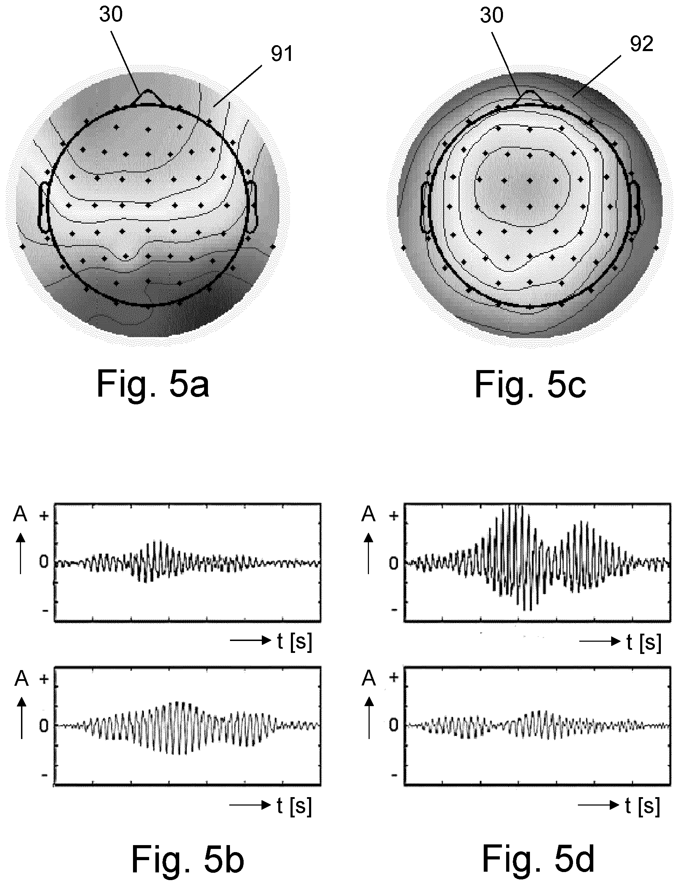

Figures 5a and 5c show an example wherein the spatio-temporal filtering method Common Spatial Patterns, CSP, or its multi-frequency extension Filter Bank Common Spatial Pattern is used to track changes in the band power of a selected frequency range or frequency ranges resulting from the activity in a certain area of the cortex. Due to an eigenvalue comparison of examples of trials indicating different levels of activity in that cortical area, a linear combination of channels is derived, maximizing the band power difference between these levels. Two exemplary linear distributions 90 and 91 are depicted here, forming a so-called CSP pattern pair, respectively.

Figures 5b and 5d show that the band power in a certain frequency range of the EEG can reflect transient changes of cognitive states, like mental workload or the level of agreement. The linear combination of the EEG channels resulting from the CSP approach, shown in Figures 5a and 5c leads to meta-channels aggregating the activity resulting from changes in that cognitive state. Here, raw EEG was referenced to the common average and filtered between 7 and 13 Hz and CSP was applied. Amplitude changes in the activity of the meta-channel produced by a CSP pattern, illustrated here, indicates transient changes of the investigated cognitive state. Light grey reflects activity related to one cognitive state, while dark grey indicates activity related to another cognitive state.

The graphs in Figures 5b and 5d are depicted on an arbitrary amplitude, A, scale running along the vertical axis, and time t running along the horizontal axis, depicted in seconds, s.

Although just a limited number of examples of pBCI algorithms are described, it will be appreciated that these are not the only feasible pBCI algorithms or approaches, or paradigms, or pipelines that can be used.

Figure 6 illustrates in a flow-chart type diagram 90 steps of the method according to the present application, implemented and executed by an information processing device operating an ML algorithm.

In a step 91, that may be continuously operated, operational data, implicit human mental brain activity data and human mental engagement of a human participating with a particular context are acquired, as disclosed above.
In a step 92, an aspect of the context is identified from the human mental engagement with the context sensed in step 91.

In a step 93, operational data and human brain activity data with the identified aspect are selected from the data acquired.

In a step 94, from the selected implicit human brain activity data, human mental state data are assessed.

The acquired operational data and assessed human mental state data are processed in step 95, which may comprise associating operational data and human mental brain activity data, i.e. assessed human mental state data, and based on this data processing, analysis and inferencing by the ML algorithm and/or aspect(s) of the context and/or any other software executed may be controlled or adapted, as depicted in step 96.

The data processing in step 95 may comprise, among others, combining or associating operational data and human mental brain activity data, i.e. assessed human mental state data.

The methods and systems described herein can be implemented in many ways. For example, the method can be encoded on a computer-readable media, such as CD-ROMs, memory cards, optical drives, magnetic drives, flash drive, or the like. The system can be implemented on a general use computer, a specialized computing system, or implemented in hardware/firmware. These are collectively known as "information processing devices" herein.

## Claims

1. A data processing method performed by an information processing device (50) operating a machine learning algorithm (51), said machine learning algorithm (51) processing operational data (58) originating from a context and human mental brain activity data (45) relating to human participation (31, 32) with said context provided by a brain-computer interface operating a respective classifier, wherein said operational data (58) and human mental brain activity data (45) are identified by sensing (46, 47, 48) human mental engagement with an aspect of said context and wherein at least one of analysis and inferencing (52) by said machine learning algorithm (51) and aspects of said context are controlled (59) by said information processing device (50) based on said data processing (52), **characterized in that** said operational data (58) comprise at least one of sensed, perceived, obtained or otherwise identified physical data, virtual data and environmental information originating from or relating to the context which is a real-life context (10) that is a non-predefined or authentic non-virtual context or environment occurring in reality or practice, and said human mental brain activity data (45) comprise implicit human participation with said real-life context (10) provided by the brain-computer interface which is a passive brain-computer interface (35, 54) operating one or more classifiers (54) associated with different types of response or mental states of human participation, wherein said operational data (58) and said human mental brain activity data (45) processed by said machine learning algorithm (51) are identified by sensing human mental engagement with an aspect of said real-life context (10) referring to what aspect or aspects (39) of said real-life context (10) said human participation (31, 32) is momentarily implicitly involved with continuously monitored by the passive brain-computer interface (35, 54) operating a respective classifier or classifiers (54), and wherein at least one of analysis and inferencing (52) by said machine learning algorithm (51) and aspects (39) of said real-life context (10) are controlled (59) by said information processing device (50) based on said data processing (52) to impart human knowledge, intelligence, subjective interpretations and human advise about tasks, processes, devices, and information perceived from said real-life context (10).

2. The data processing method according to claim 1, wherein at least one of said analysis and inferencing (52) by said machine learning algorithm (51) and aspects (39) of said real-life context (10) are controlled by said information processing device (50) by associating said identified operational data (58) and human mental brain activity data (45).

3. The data processing method according to any of the previous claims, wherein at least one of said analysis and inferencing (52) by said machine learning algorithm (50) and aspects (39) of said real-life context (10) are adapted by said information processing device (50) based on at least one of human mental engagement with respect to respective operations and interactions in said real-life context (10) and engagement of said information processing device (50) with respective operations and interactions in said real-life context (10).

4. The data processing method according to any of the previous claims, wherein said real-life context (10) comprises a cognitive probing event.

5. The data processing method according to any of the previous claims, wherein said human brain activity data (45) comprise implicit human participation with said real-life context (10) of at least one of an individual (30) and a group of individuals (49).

6. The data processing method according to any of the previous claims, wherein human mental engagement with an aspect (39) of said real-life context (10) is identified from human bio-signal data (46, 47, 48) sensed by at least one bio-signal sensor (36, 37, 38).

7. The data processing method according to any of the previous claims, wherein operational data (58, 59), human mental brain activity data (45) and human mental engagement data (45, 46, 47, 48) are processed (52) in real-time or quasi real-time, in particular data pertaining to a time critical real-life context (10).

8. The data processing method according to any of the previous claims, wherein said operational data (58, 59) comprise at least one of physical data and virtual data originating from said real-life context (10), in particular data pertaining to technological states and technological state changes of at least one device(11-23) operating in said real-life context, in particular at least one device controlled by said information processing device (50), comprising any of device input states, device output states, device operational states, device game states, computer aided design states, computer simulated design states, computer peripheral device states, computer controlled machinery states and respective state changes.

9. The data processing method according to any of the previous claims, wherein said information processing device (50) operates a reinforcement machine learning algorithm (52), in particular a deep reinforcement machine learning algorithm, comprising a reward function, wherein said implicit human mental brain activity data (45) provide said reward function.

10. The data processing method according to any of the previous claims, wherein said machine learning algorithm comprises an Artificial General Intelligence, AGI, data processing algorithm (52).

11. The data processing method (90) according to any of the previous claims, comprising the steps of:
- identifying (92), by said information processing device (50), an aspect (39) of said real-life context (10), said aspect (90) identified from sensing (35, 54; 46, 47, 48) human mental engagement with said real-life context (10);
- acquiring (93), by said information processing device (50), operational data (58) pertaining to said identified aspect (39);
- acquiring (93), by said information processing device (50), human mental brain activity data (45) of implicit human participation with said real-life context (10) pertaining to said identified aspect (39);
- processing (95), by said machine learning algorithm (51), said acquired operational data (58) and mental state data assessed (94) from said acquired human mental brain activity data (45), and
- controlling (96), by said information processing device (50), at least one of analysis and inferencing (52) by said machine learning algorithm (51) and aspects (39) of said real-life context (10) based on said data processing (52).

12. The data processing method according to any of the previous claims, wherein said information processing device (50) operates at least one further algorithm (53) for at least one of sensing, performing, acquiring, processing, and pre-processing of at least one of brain activity data (45), operational data (58, 59), human mental engagement (54), aspect identification (39), mental state data (54), human bio-signal data (46, 47, 48) and control of said machine learning algorithm (51) and aspects (39) of said real-life context (10).

13. A program product (60), encoded with instructions stored on any of a transitory and a non-transitory medium readable by an information processing device (50), said instructions arranged to perform the data processing method according to any of the previous claims when said instructions are executed by the information processing device (50) including any of a computer and a computer application.

14. The program product according to claim 13, storing a trained machine-learning algorithm (61) trained in accordance with the data processing method according to any of the claims 1 - 12.

15. A data processing system (50), comprising means arranged for performing the data processing method according to any of the claims 1 - 12.

## Patentansprüche

1. Datenverarbeitungsverfahren, das von einer Informationsverarbeitungsvorrichtung (50) durchgeführt wird, die einen Algorithmus für maschinelles Lernen (51) betreibt, wobei dieser Algorithmus für maschinelles Lernen (51) operative Daten (58), die aus einem Kontext stammen, verarbeitet und menschliche mentale Gehirnaktivitätsdaten (45), die sich auf die menschliche Teilhabe (31, 32) an dem Kontext beziehen, und die von einer Gehirn-Computer-Schnittstelle bereitgestellt werden, die einen entsprechenden Klassifikator betreibt, wobei die operative Daten (58) und die menschlichen mentalen Gehirnaktivitätsdaten (45) durch Erfassen (46, 47, 48) menschlichen mentalen Auseinandersetzung mit einem Aspekt des Kontexts identifiziert werden, und wobei mindestens eines von Analyse und Schlussfolgerung (52) durch den Algorithmus für maschinelles Lernen (51) und Aspekte des Kontexts durch die Informationsverarbeitungsvorrichtung (50) auf der Grundlage der Datenverarbeitung (52) gesteuert (59) werden, **dadurch gekennzeichnet, dass** die operative Daten (58) mindestens eines von erfassten, wahrgenommenen, erhaltenen oder anderweitig identifizierten physischen Daten, virtuellen Daten und Umgebungsinformationen umfassen, die aus dem Kontext stammen oder sich auf den Kontext beziehen, der ein realer Kontext (10) ist, welcher ein nicht-vordefinierter oder authentischer nicht-virtueller Kontext oder eine nicht-virtuelle Umgebung ist, der/die in der Realität oder in der Praxis vorkommt, und die menschlichen mentalen Gehirnaktivitätsdaten (45) eine implizite menschliche Teilhabe an dem realen Kontext (10) umfassen, die von der Gehirn-Computer-Schnittstelle bereitgestellt wird, die eine passive Gehirn-Computer-Schnittstelle (35, 54) ist, die einen oder mehrere Klassifikatoren (54) betreibt, die an verschiedene Arten von Reaktionen oder mentalen Zuständen menschlichen Teilhabe verknüpft sind, wobei die operative Daten (58) und die menschlichen mentalen Gehirnaktivitätsdaten (45), die von dem Algorithmus für maschinelles Lernen (51) verarbeitet werden, durch Erfassen der menschlichen mentalen Auseinandersetzung mit einem Aspekt des realen Kontexts (10) identifiziert werden, der sich darauf bezieht, mit welchem Aspekt oder welchen Aspekten (39) des realen Kontexts (10) die menschliche Teilhabe (31, 32) momentan implizit beschäftigt ist, kontinuierlich überwacht von der passiven Gehirn-Computer-Schnittstelle (35, 54), die einen entsprechenden Klassifikator oder Klassifikatoren (54) betreibt, und wobei mindestens eine der Analysen und Schlussfolgerungen (52) durch den Algorithmus für maschinelles Lernen (51) und Aspekte (39) des realen Kontexts (10) durch die Informationsverarbeitungsvorrichtung (50) auf der Grundlage der Datenverarbeitung (52) gesteuert (59) werden, um menschliches Wissen, Intelligenz, subjektive Interpretationen und menschliche Ratschläge über Aufgaben, Prozesse, Geräte und Informationen, die aus dem realen Kontext (10) wahrgenommen werden, zu vermitteln.

2. Datenverarbeitungsverfahren nach Anspruch 1, wobei mindestens eine der Analysen und Schlussfolgerungen (52) durch den Algorithmus für maschinelles Lernen (51) und Aspekte (39) des realen Kontexts (10) durch die Informationsverarbeitungsvorrichtung (50) gesteuert werden, durch verknüpfen der identifizierten operativen Daten (58) und der menschlichen mentalen Gehirnaktivitätsdaten (45).

3. Datenverarbeitungsverfahren nach einem der vorhergehenden Ansprüche, wobei mindestens eine der Analysen und Schlussfolgerungen (52) durch den Algorithmus für maschinelles Lernen (50) und Aspekte (39) des realen Kontexts (10) durch die Informationsverarbeitungsvorrichtung (50) angepasst werden auf der Grundlage von mindestens eines der menschlicher mentaler Auseinandersetzung in Bezug auf entsprechende Operationen und Interaktionen in dem realen Kontext (10) und Einsatz der Informationsverarbeitungsvorrichtung (50) mit entsprechenden Operationen und Interaktionen in dem realen Kontext (10).

4. Datenverarbeitungsverfahren nach einem der vorhergehenden Ansprüche, wobei der reale Kontext (10) ein kognitives Sondierungsereignis umfasst.

5. Datenverarbeitungsverfahren nach einem der vorhergehenden Ansprüche, wobei die menschlichen Gehirnaktivitätsdaten (45) die implizite menschliche Teilhabe an dem realen Kontext (10) mindestens eines von einem Individuum (30) und einer Gruppe von Individuen (49) umfassen.

6. Datenverarbeitungsverfahren nach einem der vorhergehenden Ansprüche, wobei menschliche mentale Auseinandersetzung mit einem Aspekt (39) des realen Kontexts (10) anhand von menschlichen Biosignaldaten (46, 47, 48) identifiziert wird, die von mindestens einem Biosignalsensor (36, 37, 38) erfasst werden.

7. Datenverarbeitungsverfahren nach einem der vorhergehenden Ansprüche, wobei operative Daten (58, 59), menschlichen Gehirnaktivitätsdaten (45) und menschlichen mentalen Auseinandersetzungsdaten (45, 46, 47, 48) in Echtzeit oder Quasi-Echtzeit verarbeitet (52) werden, insbesondere Daten, die sich auf einen zeitkritischen realen Kontext (10) beziehen.

8. Datenverarbeitungsverfahren nach einem der vorhergehenden Ansprüche, wobei die operative Daten (58, 59) mindestens eines von physikalischen Daten und virtuellen Daten umfassen, die aus dem realen Kontext (10) stammen, insbesondere Daten, die sich auf technologische Zustände und technologische Zustandsänderungen von mindestens einer Vorrichtung (11-23) beziehen, die in dem realen Kontext arbeitet, insbesondere mindestens eine von der Informationsverarbeitungsvorrichtung (50) gesteuerte Vorrichtung, und die beliebig viele des Folgenden umfassen: Vorrichtungseingangszustände, Vorrichtungsausgangszustände, Vorrichtungsbetriebszustände, Vorrichtungsspielzustände, computergestützte Entwurfszustände, computersimulierte Entwurfszustände, Computerperipherie-vorrichtungszustände, computergesteuerte Maschinenzustände und entsprechende Zustandsänderungen.

9. Datenverarbeitungsverfahren nach einem der vorhergehenden Ansprüche, wobei die Informationsverarbeitungsvorrichtung (50) einen Algorithmus für maschinelles bestärkendes Lernen (52), insbesondere einen Algorithmus für tiefes maschinelles bestärkendes Lernen, betreibt, der eine Belohnungsfunktion umfasst, wobei die impliziten menschlichen mentalen Gehirnaktivitätsdaten (45) die Belohnungsfunktion liefern.

10. Datenverarbeitungsverfahren nach einem der vorhergehenden Ansprüche, wobei der Algorithmus für maschinelles Lernen einen Datenverarbeitungsalgorithmus (52) für künstliche allgemeine Intelligenz, AGI, umfasst.

11. Datenverarbeitungsverfahren (90) nach einem der vorhergehenden Ansprüche, umfassend die Schritte:
- Identifizieren (92), durch die Informationsverarbeitungsvorrichtung (50), eines Aspekts (39) des realen Kontexts (10), wobei der Aspekt (90) durch Erfassen (35, 54; 46, 47, 48) menschlichen mentalen Auseinandersetzung mit dem realen Kontext (10) identifiziert wird;
- Erwerben (93), durch die Informationsverarbeitungsvorrichtung (50), von operativen Daten (58) die sich auf den identifizierten Aspekt (39) beziehen;
- Erwerben (93), durch die Informationsverarbeitungsvorrichtung (50), von menschlichen mentalen Gehirnaktivitätsdaten (45) der impliziten menschlichen Teilhabe an dem realen Kontext (10), die sich auf den identifizierten Aspekt (39) beziehen;
- Verarbeiten (95), durch den Algorithmus für maschinelles Lernen (51), der erworben operativen Daten (58) und mentalen Zustandsdaten die aus den erworben menschlichen mentalen Gehirnaktivitätsdaten (45) ermittelt (94) wurden, und
- Steuern (96), durch die Informationsverarbeitungsvorrichtung (50), von mindestens einer von Analyse und Schlussfolgerung (52) durch den Algorithmus für maschinelles Lernen (51) und Aspekten (39) des realen Kontexts (10), basierend auf der Datenverarbeitung (52).

12. Datenverarbeitungsverfahren nach einem der vorhergehenden Ansprüche, wobei die Informationsverarbeitungsvorrichtung (50) mindestens einen weiteren Algorithmus (53) betreibt zur Erfassung, Durchführung, Ermittlung, Verarbeitung und Vorverarbeitung von mindestens einem von Gehirnaktivitätsdaten (45), operative Daten (58, 59), menschliche mentale Auseinandersetzung (54), Aspekterkennung (39), mentale Zustandsdaten (54), menschliche Biosignaldaten (46, 47, 48) und Steuerung des Algorithmus für maschinelles Lernen (51) und Aspekte (39) des realen Kontexts (10).

13. Programmprodukt (60), das mit Instruktionen kodiert ist, die auf einem transitorischen oder einem nicht-transitorischen Medium gespeichert sind, das von einer Informationsverarbeitungsvorrichtung (50) gelesen werden kann, wobei die Instruktionen so angeordnet sind, dass sie das Datenverarbeitungsverfahren gemäß einem der vorhergehenden Ansprüche durchführen, wenn die Instruktionen von der Informationsverarbeitungsvorrichtung (50) ausgeführt werden, einschließlich eines von einem Computer und einer Computeranwendung.

14. Programmprodukt nach Anspruch 13, das einen trainierten Algorithmus für maschinelles Lernen (61) speichert, der gemäß dem Datenverarbeitungsverfahren nach einem der Ansprüche 1 bis 12 trainiert wurde

15. Datenverarbeitungssystem (50), das Mittel zur Durchführung des Datenverarbeitungsverfahrens nach einem der Ansprüche 1 bis 12 enthält.

## Revendications

1. Procédé de traitement de données réalisé par un dispositif de traitement de l'information (50) exploitant un algorithme d'apprentissage automatique (51), ledit algorithme d'apprentissage automatique (51) traitant des données opérationnelles (58) provenant d'un contexte et des données d'activité cérébrale mentale humaine (45) relatives à la participation humaine (31, 32) à ledit contexte, fournies par une interface cerveau-ordinateur exploitant un classificateur respectif, lesdites données opérationnelles (58) et données d'activité cérébrale mentale humaine (45) étant identifiées par la détection (46, 47, 48) de l'engagement mental humain avec un aspect dudit contexte et où au moins une des opérations d'analyse et d'inférence (52) par ledit algorithme d'apprentissage automatique (51) et des aspects dudit contexte sont contrôlés (59) par ledit dispositif de traitement de l'information (50) sur la base de ce traitement de données (52), **caractérisé en ce que** lesdites données opérationnelles (58) comprennent au moins l'une des données physiques, virtuelles et informations environnementales détectées, perçues, obtenues ou autrement identifiées, provenant de ou se rapportant au contexte qui est un contexte de vie réelle (10), c'est-à-dire un contexte non prédéfini ou authentique non virtuel se produisant dans la réalité ou la pratique, et lesdites données d'activité cérébrale mentale humaine (45) comprennent une participation humaine implicite avec ledit contexte de vie réelle (10) fournie par l'interface cerveau-ordinateur qui est une interface cerveau-ordinateur passive (35, 54) exploitant un ou plusieurs classificateurs (54) associés à différents types de réponses ou états mentaux de la participation humaine, lesdites données opérationnelles (58) et lesdites données d'activité cérébrale mentale humaine (45) traitées par ledit algorithme d'apprentissage automatique (51) étant identifiées par la détection de l'engagement mental humain avec un aspect dudit contexte de vie réelle (10) se référant à quel aspect ou aspects (39) dudit contexte de vie réelle (10) ladite participation humaine (31, 32) est momentanément impliquée de manière implicite, surveillée en continu par l'interface cerveau-ordinateur passive (35, 54) exploitant un ou plusieurs classificateurs respectifs (54), et où au moins une des opérations d'analyse et d'inférence (52) par ledit algorithme d'apprentissage automatique (51) et des aspects (39) dudit contexte de vie réelle (10) sont contrôlés (59) par ledit dispositif de traitement de l'information (50) sur la base de ce traitement de données (52) pour transmettre des connaissances humaines, une intelligence, des interprétations subjectives et des conseils humains sur des tâches, des processus, des dispositifs et des informations perçues dudit contexte de vie réelle (10).

2. Le procédé de traitement de données selon la revendication 1, où au moins une des opérations d'analyse et d'inférence (52) par ledit algorithme d'apprentissage automatique (51) et des aspects (39) dudit contexte de vie réelle (10) sont contrôlés par ledit dispositif de traitement de l'information (50) en associant lesdites données opérationnelles identifiées (58) et les données d'activité cérébrale mentale humaine (45).

3. Le procédé de traitement de données selon l'une des revendications précédentes, où au moins une des opérations d'analyse et d'inférence (52) par ledit algorithme d'apprentissage automatique (50) et des aspects (39) dudit contexte de vie réelle (10) sont adaptées par ledit dispositif de traitement de l'information (50) sur la base d'au moins l'un de l'engagement mental humain par rapport à des opérations respectives et des interactions dans ledit contexte de vie réelle (10) et de l'engagement dudit dispositif de traitement de l'information (50) avec des opérations respectives et des interactions dans ledit contexte de vie réelle (10).

4. Le procédé de traitement de données selon l'une des revendications précédentes, où ledit contexte de vie réelle (10) comprend un événement de sondage cognitif.

5. Le procédé de traitement de données selon l'une des revendications précédentes, où lesdites données d'activité cérébrale humaine (45) comprennent une participation humaine implicite avec ledit contexte de vie réelle (10) d'au moins un individu (30) et un groupe d'individus (49).

6. Le procédé de traitement de données selon l'une des revendications précédentes, où l'engagement mental humain avec un aspect (39) dudit contexte de vie réelle (10) est identifié à partir de données bio-signal humain (46, 47, 48) détectées par au moins un capteur de bio-signal (36, 37, 38).

7. Le procédé de traitement de données selon l'une des revendications précédentes, où les données opérationnelles (58, 59), les données d'activité cérébrale mentale humaine (45) et les données d'engagement mental humain (45, 46, 47, 48) sont traitées (52) en temps réel ou quasi-temps réel, en particulier des données relatives à un contexte de vie réelle critique en temps (10).

8. Le procédé de traitement de données selon l'une des revendications précédentes, où lesdites données opérationnelles (58, 59) comprennent au moins des données physiques et des données virtuelles provenant dudit contexte de vie réelle (10), en particulier des données relatives aux états technologiques et aux changements d'états technologiques d'au moins un dispositif (11-23) fonctionnant dans ledit contexte de vie réelle, en particulier au moins un dispositif contrôlé par ledit dispositif de traitement de l'information (50), comprenant l'une des états d'entrée de dispositif, états de sortie de dispositif, états opérationnels de dispositif, états de jeu de dispositif, états de conception assistée par ordinateur, états de conception simulée par ordinateur, états de périphérique d'ordinateur, états de machinerie contrôlée par ordinateur et changements d'états respectifs.

9. Le procédé de traitement de données selon l'une des revendications précédentes, où ledit dispositif de traitement de l'information (50) exploite un algorithme d'apprentissage automatique par renforcement (52), en particulier un algorithme d'apprentissage automatique par renforcement profond, comprenant une fonction de récompense, lesdites données d'activité cérébrale mentale humaine implicites (45) fournissant ladite fonction de récompense.

10. Le procédé de traitement de données selon l'une des revendications précédentes, où ledit algorithme d'apprentissage automatique comprend un algorithme de traitement de données d'Intelligence Générale Artificielle, AGI, (52).

11. Le procédé de traitement de données (90) selon l'une des revendications précédentes, comprenant les étapes de :
- identification (92), par ledit dispositif de traitement de l'information (50), d'un aspect (39) dudit contexte de vie réelle (10), ledit aspect (90) identifié à partir de la détection (35, 54 ; 46, 47, 48) de l'engagement mental humain avec ledit contexte de vie réelle (10) ;
- acquisition (93), par ledit dispositif de traitement de l'information (50), des données opérationnelles (58) se rapportant à ledit aspect identifié (39) ;
- acquisition (93), par ledit dispositif de traitement de l'information (50), des données d'activité cérébrale mentale humaine (45) de participation humaine implicite avec ledit contexte de vie réelle (10) se rapportant à ledit aspect identifié (39) ;
- traitement (95), par ledit algorithme d'apprentissage automatique (51), desdites données opérationnelles acquises (58) et des données d'état mental évaluées (94) à partir desdites données d'activité cérébrale mentale humaine acquises (45), et
- contrôle (96), par ledit dispositif de traitement de l'information (50), d'au moins une des opérations d'analyse et d'inférence (52) par ledit algorithme d'apprentissage automatique (51) et des aspects (39) dudit contexte de vie réelle (10) sur la base de ce traitement de données (52).

12. Le procédé de traitement de données selon l'une des revendications précédentes, où ledit dispositif de traitement de l'information (50) exploite au moins un autre algorithme (53) pour au moins une des opérations de détection, de réalisation, d'acquisition, de traitement et de prétraitement d'au moins l'une des données d'activité cérébrale (45), données opérationnelles (58, 59), engagement mental humain (54), identification d'aspect (39), données d'état mental (54), données bio-signal humain (46, 47, 48) et contrôle dudit algorithme d'apprentissage automatique (51) et des aspects (39) dudit contexte de vie réelle (10).

13. Un produit programme (60), codé avec des instructions stockées sur un support lisible, transitoire ou non transitoire, par un dispositif de traitement de l'information (50), lesdites instructions étant disposées pour réaliser le procédé de traitement de données selon l'une des revendications précédentes lorsque lesdites instructions sont exécutées par le dispositif de traitement de l'information (50) incluant tout ordinateur et toute application informatique.

14. Le produit programme selon la revendication 13, stockant un algorithme d'apprentissage automatique formé (61) formé conformément au procédé de traitement de données selon l'une des revendications 1 à 12.

15. Un système de traitement de données (50), comprenant des moyens agencés pour réaliser le procédé de traitement de données selon l'une des revendications 1 à 12.
